# EUROPÄISCHE PATENTANMELDUNG

(11) **EP 4 620 411 A2**
(43) Veröffentlichungstag der Anmeldung: **24.09.2025**
(21) Anmeldenummer: 25194507.7
(22) Anmeldetag: 27.09.2021
(51) Int. Cl.: A61B 17/28

(54) **CHIRURGISCHES INSTRUMENT MIT UMLAUFSPERRE**

(30) Priorität: 29.09.2020 DE 102020212252
(62) Teilanmeldung aus: 21783253.4
(71) Anmelder: Aesculap AG, 78532 Tuttlingen (DE)
(72) Erfinder: Hafner, Nikolaus, 78532 Tuttlingen (DE); Herner, Eugen, 78054 Villingen-Schwenningen (DE); Kizenberger, Hannes, 78194 Immendingen (DE); Walberg, Erik, 86150 Augsburg (DE)
(74) Vertreter: Patentanwälte Ruff, Wilhelm, Beier, Dauster & Partner mbB

(57) **Zusammenfassung**

2.1 Ein chirurgisches Instrument mit zwei relativ zueinander beweglichen Greifschenkeln, sowie mit einer Umlaufsperre, die dazu vorgesehen ist, die beiden Greifschenkel relativ zueinander lösbar zu arretieren, wobei die Umlaufsperre im Bereich des einen Greifschenkels eine weibliche Arretieranordnung und im Bereich des anderen Greifschenkels eine männliche Arretieranordnung aufweist, wobei die weibliche Arretieranordnung eine mechanische Steuervorrichtung zum Arretieren und Freigeben eines Arretierabschnittes der männlichen Arretieranordnung aufweist, ist bekannt.

2.2 Erfindungsgemäß ist der männlichen Arretieranordnung eine mechanische Steueranordnung zugeordnet, die dazu vorgesehen ist, den Arretierabschnitt der männlichen Arretieranordnung aus einer eine Ruhestellung definierenden Endlage in eine eine Funktionsstellung definierende Endlage und umgekehrt aus der die Funktionsstellung definierenden Endlage in die die Ruhestellung definierende Endlage zu verlagern, und dass die Steueranordnung Sicherungsmittel aufweist, die dazu vorgesehen sind, den Arretierabschnitt in beiden Endlagen zu sichern.

2.3 Einsatz für offen chirurgische Instrumente.

## Beschreibung

Die Erfindung betrifft ein chirurgisches Instrument mit zwei relativ zueinander beweglichen Greifschenkeln, sowie mit einer Umlaufsperre, die dazu vorgesehen ist, die beiden Greifschenkel relativ zueinander lösbar zu arretieren, wobei die Umlaufsperre im Bereich des einen Greifschenkels eine weibliche Arretieranordnung und im Bereich des anderen Greifschenkels eine männliche Arretieranordnung aufweist, wobei die weibliche Arretieranordnung eine mechanische Steuervorrichtung zum Arretieren und Freigeben eines Arretierabschnittes der männlichen Arretieranordnung aufweist.

Ein derartiges chirurgisches Instrument ist aus der DE 10 2016 118 199 A1 bekannt. Das bekannte chirurgische Instrument ist als Klemminstrument ausgeführt, bei dem die beiden Greifschenkel sich distal in Klemmbacken fortsetzen, die durch Relativbewegungen der Greifschenkel zueinander für eine Klemmung zusammengeführt oder auseinanderbewegt werden. Das bekannte Klemminstrument weist eine Umlaufsperre auf, die die Greifschenkel lösbar relativ zueinander arretiert, so dass ein Klemmzustand der Klemmbacken aufrechterhalten werden kann. Die Umlaufsperre weist eine männliche Arretieranordnung an dem einen Greifschenkel und eine weibliche Arretieranordnung an dem in einer Schwenkebene der Greifschenkel gegenüberliegenden Greifschenkel auf. Die männliche Arretieranordnung ist mit einem zu dem gegenüberliegenden Greifschenkel abragenden Arretierabschnitt versehen, der längs des Greifschenkels translatorisch verschiebbar gelagert ist. Die an dem gegenüberliegenden Greifschenkel vorgesehene weibliche Arretieranordnung ist zur formschlüssig verrastenden Aufnahme des Arretierabschnittes der männlichen Arretieranordnung vorgesehen und weist eine längs des zugehörigen Greifschenkels linearbeweglich gelagerte Steuerkulisse auf, die den Arretierabschnitt der gegenüberliegenden männlichen Arretieranordnung nach Art eines Kugelschreiberprinzips aufnimmt, sichert und wieder freigibt abhängig von entsprechenden Relativbewegungen der beiden Greifschenkel. Bei einer Schließbewegung der Greifschenkel relativ zueinander taucht der Arretierabschnitt in die federbelastet bewegliche Steuerkulisse ein. Eine anschließende Entlastung der auf die beiden Greifschenkel ausgeübten manuellen Schließbewegung führt zu einer Verrastung des Arretierabschnittes innerhalb der Steuerkulisse der weiblichen Arretieranordnung. Ein erneutes Zusammendrücken der Greifschenkel durch eine manuelle Belastung führt zu einem Lösen des Arretierabschnittes aus der Steuerkulisse des weiblichen Arretierabschnittes.

Aufgabe der Erfindung ist es, ein chirurgisches Instrument der eingangs genannten Art zu schaffen, das eine besonders zuverlässige und sichere Arretierung und Freigabe der beiden Greifschenkel relativ zueinander ermöglicht.

Diese Aufgabe wird dadurch gelöst, dass der männlichen Arretieranordnung eine mechanische Steueranordnung zugeordnet ist, die dazu vorgesehen ist, den Arretierabschnitt der männlichen Arretieranordnung aus einer eine Ruhestellung definierenden Endlage in eine eine Funktionsstellung definierende Endlage und umgekehrt aus der die Funktionsstellung definierenden Endlage in die die Ruhestellung definierende Endlage zu verlagern, und dass die Steueranordnung Sicherungsmittel aufweist, die dazu vorgesehen sind, den Arretierabschnitt in beiden Endlagen zu sichern. Die erfindungsgemäße Lösung gewährleistet, dass der Arretierabschnitt sich immer in einer definierten Endlage befindet. Undefinierte Zwischenlagen, die beim Stand der Technik dazu führen konnten, dass keine zuverlässige Arretierung der Umlaufsperre erzielt werden konnte, werden durch die Erfindung vermieden. Die mechanische Steueranordnung kann eine Federeinrichtung aufweisen, um den Arretierabschnitt der männlichen Arretieranordnung in eine Endlage zu überführen. Die Federeinrichtung kann auch als Sicherungsmittel dienen, um den Arretierabschnitt in der Endlage zu sichern. Die Sicherungsmittel sind vorzugsweise mechanisch ausgeführt. Alternativ können diese auch elektromagnetisch oder permanentmagnetisch wirksam sein. Der Arretierabschnitt kann translatorisch oder rotatorisch zwischen seinen Endlagen verlagerbar sein. Die erfindungsgemäße Lösung eignet sich besonders vorteilhaft für offen chirurgische Instrumente. Vorzugsweise weist das chirurgische Instrument Klemmbacken auf, die mittels der Greifschenkel beweglich sind. Es ist möglich, die Klemmbacken mit Strom zu beaufschlagen. Die mechanische Steueranordnung kann durch einen elektrischen Antrieb unterstützt sein, um eine Aktivierung oder Deaktivierung der Umlaufsperre zu erzielen.

In Ausgestaltung der Erfindung ist eine manuell bedienbare Betätigungseinrichtung vorgesehen, die mit wenigstens einem Sicherungsmittel in Wirkverbindung ist, um das wenigstens eine Sicherungsmittel für eine Freigabe einer Verlagerbarkeit des Arretierabschnittes anzusteuern. Dadurch ist es möglich, die mechanische Steueranordnung bei Bedarf durch einen entsprechenden Anwender manuell zu aktivieren. Ohne manuelle Aktivierung bleibt der männliche Arretierabschnitt in einer definierten Endlage, sodass entweder eine Arretierung mit der weiblichen Arretieranordnung möglich ist, oder das eine Interaktion mit der weiblichen Arretieranordnung ausgeschlossen ist abhängig davon, ob der männliche Arretierabschnitt sich in der die Funktionsstellung (Arretierstellung) definierenden Endlage oder in der die Ruhestellung definierenden Endlage befindet.

In weiterer Ausgestaltung der Erfindung ist der Arretierabschnitt für eine Verlagerung zwischen den beiden Endlagen längs einer Linearführung translatorisch geführt. Die Linearführung erstreckt sich vorzugsweise in Längsrichtung des Greifschenkels entlang eines dem gegenüberliegenden Greifschenkel zugewandten Randbereichs dieses Greifschenkels. Die Linearführung ist vorzugsweise geradlinig ausgeführt, kann sich alternativ aber im Bereich des Greifschenkels auch längs einer Kurvenbahn erstrecken. Die Linearführung kann einstückig in dem Greifschenkel integriert sein oder in einem separat hergestellten Trägerteil vorgesehen sein, das in einem Betriebszustand mit dem Greifschenkel fest verbunden ist.

In weiterer Ausgestaltung der Erfindung verläuft die Linearführung längs einer Kurvenbahn, insbesondere längs eines Kreisbogens. Die Kurvenbahn, insbesondere der Kreisbogen, erstreckt sich in der Schwenkebene des Greifschenkels. Die Linearführung ist vorzugsweise so ausgeführt, dass der Arretierabschnitt in seiner die Ruhestellung definierenden Endlage innerhalb entsprechender Außenkonturen des Greifschenkels positioniert ist, so dass der Arretierabschnitt in dieser Ruhestellung nicht über den Greifschenkel nach außen abragt. Hierdurch wird zuverlässig vermieden, dass eine Bedienperson insbesondere mit einem Handschuh bei der Handhabung des chirurgischen Instrumentes an dem Arretierabschnitt hängen bleibt.

In weiterer Ausgestaltung der Erfindung ist wenigstens ein Sicherungsmittel als kraftschlüssig wirksame Sicherungseinheit ausgeführt. Die kraftschlüssig wirksame Sicherungseinheit weist vorzugsweise zueinander komplementäre Reibungsabschnitte ortsfest im Bereich des Greifschenkels einerseits und beweglich am Arretierabschnitt andererseits auf, insbesondere in Form einer noppenartigen Profilierung einerseits und einer vertieften Profilierung andererseits. In vorteilhafter Weise ist die kraftschlüssig wirksame Sicherungseinheit kraftbegrenzt lösbar gestaltet. Die Sicherungseinheit ist demzufolge kraftbegrenzt wirksam. Sobald eine auf den Arretierabschnitt ausgeübte Verlagerungskraft höher ist als die Rückhaltekraft der Sicherungseinheit in der entsprechenden Endlage wird der Arretierabschnitt für eine Überführung in die entsprechend andere Endlage freigegeben. Die kraftschlüssig wirksame Sicherungseinheit kann auch einen Permanentmagneten oder einen Elektromagneten aufweisen.

In weiterer Ausgestaltung der Erfindung ist wenigstens ein Sicherungsmittel als formschlüssig wirksame Sicherungseinheit ausgeführt. Die formschlüssig wirksame Sicherungseinheit weist einen Lösemechanismus auf, der den Arretierabschnitt bei Bedarf freigibt.

In weiterer Ausgestaltung der Erfindung ist der formschlüssig wirksamen Sicherungseinheit die manuell bedienbare Betätigungseinrichtung zugeordnet, um die Sicherungseinheit aus einer Sicherungsstellung in eine Freigabestellung zu überführen. Die manuell bedienbare Betätigungseinrichtung kann einen Lösemechanismus im Sinne der zuvor beschriebenen Ausgestaltung aufweisen. Die manuell bedienbare Betätigungseinrichtung weist vorzugsweise wenigstens ein Bedienelement auf, das ergonomisch günstig im Bereich von einem der Greifschenkel positioniert ist. Ergänzend oder alternativ ist es verschmutzungsunempfindlich ausgeführt. Vorzugsweise ist die manuell bedienbare Betätigungseinrichtung derart ausgeführt, dass sie von Linkshändern wie Rechtshändern gleichermaßen problemlos und ergonomisch günstig bedienbar ist.

In weiterer Ausgestaltung der Erfindung ist die formschlüssig wirksame Sicherungseinheit spiegelsymmetrisch relativ zu einer Verlagerungsebene der Sicherungseinheit gestaltet, wobei die Sicherungseinheit insbesondere auf gegenüberliegenden Seiten des Greifschenkels jeweils ein elastisch bewegliches Rastelement aufweist, die mit ortsfesten Rastaussparungen im Bereich des Greifschenkels zusammenwirken. Die Sicherungseinheit ist vorzugsweise spiegelsymmetrisch zu einer Schwenkebene der Greifschenkel gestaltet. Die spiegelsymmetrische Anordnung von zwei elastisch beweglichen Rastelementen ermöglicht eine besonders sichere Arretierung des translatorisch beweglichen Arretierabschnittes in der entsprechenden Endlage. Die Rastelemente sind vorzugsweise mit der manuell bedienbaren Betätigungseinrichtung in Wirkverbindung.

In weiterer Ausgestaltung der Erfindung ist der Arretierabschnitt mittels einer Drehlagerung rotatorisch geführt. Die Drehlagerung weist vorzugsweise eine Drehachse auf, die sich in Längsrichtung des Greifschenkels und damit zumindest weitgehend parallel zu einem zu dem gegenüberliegenden Greifschenkel benachbarten Randbereich des Greifschenkels erstreckt. Die rotatorische Führung des Arretierabschnittes ermöglicht es, den Arretierabschnitt in der Funktionsstellung von dem Greifschenkel abragend zu positionieren, und den Arretierabschnitt in der Ruhelage innerhalb einer Außenkontur des zugehörigen Greifschenkels zu parken.

In weiterer Ausgestaltung der Erfindung weist der Greifschenkel wenigstens eine Aussparung auf, in der der Arretierabschnitt in der Ruhelage aufgenommen ist. Vorzugsweise ist der Arretierabschnitt in der Aussparung bündig aufgenommen. Die wenigstens eine Aussparung kann als einseitig zumindest abschnittsweise geschlossene Vertiefung, insbesondere in Form einer Tasche oder einer ähnlichen, nicht durchgängigen Vertiefung, oder auch als zu beiden Seiten des Greifschenkels offener Durchtritt ausgeführt sein. Je nach Ausgestaltung der wenigstens einen Aussparung ist somit eine Rotation des Arretierabschnittes um weniger als 360° oder um 360° und mehr möglich. Dadurch, dass der Arretierabschnitt in eine Ruhelage verdreht werden kann, in der der Arretierabschnitt nicht über den zugehörigen Greifschenkel absteht, wird es zuverlässig vermieden, dass eine Bedienperson unbeabsichtigt an dem Arretierabschnitt hängen bleibt, insbesondere mittels eines Handschuhs.

In weiterer Ausgestaltung der Erfindung weist der Greifschenkel einen auf Außenabmessungen des Arretierabschnittes abgestimmten Durchtritt auf, der derart gestaltet ist, dass der Arretierabschnitt in entgegengesetzten Drehrichtungen durch den Durchtritt hindurch rotieren kann. Dadurch kann der Arretierabschnitt nicht nur in einer Drehrichtung, sondern in beiden Drehrichtungen aus der Ruhestellung in die Funktionsstellung oder umgekehrt überführt werden. Diese Ausgestaltung ist in ergonomischer Sicht vorteilhaft, da sowohl für Linkshänder als auch für Rechtshänder eine einfache Bedienung möglich ist, um den Arretierabschnitt zu verdrehen.

In weiterer Ausgestaltung der Erfindung ist dem Arretierabschnitt eine mechanische Zwangssteuereinrichtung zugeordnet, die eine zwangsläufige Überführung des Arretierabschnittes aus einer Zwischenlage in eine Endlage vornimmt. Die mechanische Zwangssteuereinrichtung ermöglicht es bei rotatorischer Führung des Arretierabschnittes, dass der Arretierabschnitt bei entsprechender Aktivierung der Zwangssteuereinrichtung selbsttätig aus einer Zwischenlage in die entsprechende Endlage gesteuert wird, ohne dass dies durch eine manuelle Betätigung erfolgt. Diese Ausgestaltung vereinfacht für eine Bedienperson die Überführung des Arretierabschnittes in die entsprechende Ruhelage oder die entsprechende Funktionslage. Fehlfunktionen oder undefinierte Zwischenstellungen werden hierdurch zuverlässig vermieden. Die quasi selbsttätige Überführung in die entsprechende Endlage reduziert zudem den Betätigungsaufwand der entsprechenden Bedienperson, wodurch die Bedienung des chirurgischen Instrumentes vereinfacht ist.

In weiterer Ausgestaltung der Erfindung ist die Zwangssteuereinrichtung in mechanischer Wirkverbindung zwischen den Arretierabschnitt und ein manuell bewegliches Betätigungselement derart zwischengeschaltet, dass eine beginnende Bewegung des Betätigungselements zu einer Aktivierung der Zwangssteuereinrichtung führt. Diese Ausgestaltung benötigt ein manuell bewegliches Betätigungselement, das initial eine Bewegung in die Zwangssteuereinrichtung einleitet, die dann aktiviert ist und die gewünschte Verlagerung des Arretierabschnittes in die entsprechende Endlage vornimmt. Das manuell bewegliche Betätigungselement ist vorzugsweise ein rotatorisch im Bereich des Greifschenkels gelagertes Betätigungselement, insbesondere ein Drehrad, das mit Griffflächen, insbesondere in Form einer Riffelung, im Bereich seines Außenumfangs versehen ist, um die Bedienung des Drehrades zu vereinfachen. Das Betätigungselement ist Teil der zuvor beschriebenen Betätigungseinrichtung.

In weiterer Ausgestaltung der Erfindung weist die Zwangssteuereinrichtung eine Verzögerungseinheit auf, die eine bewegungsverzögerte Drehmomentübertragung von dem Betätigungselement auf den Arretierabschnitt bewirkt. Hierdurch wird ein begrenzter Freilauf erzielt, der vorzugsweise in einem Drehwinkelbereich zwischen 5° und 85° liegt. In diesem Drehwinkelbereich bewirkt eine Betätigung des Betätigungselements noch keine Aktivierung der Zwangssteuereinrichtung und insbesondere auch keine Überführung des Arretierabschnittes aus der einen Endlage in die andere Endlage. Die verzögerte Reaktion auf den Arretierabschnitt ermöglicht eine Sicherung des Arretierabschnittes gegen eine unbeabsichtigte Rotation aufgrund auftretender Querkräfte. Auf den Arretierabschnitt wirkende Querkräfte beeinträchtigen demzufolge die jeweilige Endlage des Arretierabschnittes nicht. Diese Ausgestaltung ist zudem vorteilhaft, da unbeabsichtigte, kleinere Drehbewegungen des Betätigungselements keine Auswirkungen auf den Sperr- oder Freigabezustand der Umlaufsperre haben.

In weiterer Ausgestaltung der Erfindung weist die Zwangssteuereinrichtung mehrere relativ zueinander koaxial drehbewegliche Funktionskomponenten auf, die mit in Drehrichtung wirksamen Steuerkonturen versehen sind, die für eine zwangsläufige Überführung des Arretierabschnittes in eine Endlage miteinander zusammenwirken. Die Zwangssteuereinrichtung ist demzufolge rotatorisch wirksam, wobei die verschiedenen Funktionskomponenten einander vorzugsweise in Längsrichtung der Drehachse zumindest teilweise überlagern, wodurch sich eine platzsparende Anordnung der Funktionskomponenten der Zwangssteuereinrichtung ergibt. Die Steuerkonturen sind vorzugsweise an einem Innenumfang und/oder an einem Außenumfang der entsprechenden Funktionskomponenten vorgesehen, um in gewünschter Weise in Umfangsrichtung miteinander zusammenzuwirken.

In weiterer Ausgestaltung der Erfindung weist die Zwangssteuereinrichtung wenigstens eine eine Aktivierungskraft auf eine Funktionskomponente der Zwangssteuereinrichtung aufbringende Federeinrichtung auf. Die Federeinrichtung weist vorteilhaft eine permanente Vorspannung auf. Die Federeinrichtung wirkt als Federantrieb, um eine eingeleitete Drehbewegung auf eine entsprechende Funktionskomponente selbsttätig - das heißt ohne manuelles Zutun - zu vollenden. Die Federeinrichtung kann als Konstantkraftfeder oder als progressiv wirksame Federeinrichtung mit einer Federrate vorzugsweise aus einem Bereich zwischen 0,1 N/mm und 10 N/mm ausgeführt sein.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
Fig. 1 zeigt in einer Draufsicht eine erste Ausführungsform eines erfindungsgemäßen chirurgischen Instrumentes,
Fig. 2 in vergrößerter Darstellung einen Teilbereich des chirurgischen Instrumentes gemäß Fig. 1,
Fig. 3 einen Teilbereich einer weiteren Ausführungsform eines erfindungsgemäßen chirurgischen Instrumentes ähnlich Fig. 2,
Fig. 4 in schematisch vergrößerter Schnittdarstellung eine manuell bedienbare, formschlüssig wirksame Sicherungseinheit einer Umlaufsperre für das chirurgische Instrument nach den Fig. 1 und 2,
Fig. 5 die Sicherungseinheit gemäß Fig. 4 unter Weglassung einer ortsfesten Linearführung innerhalb eines Greifschenkels des chirurgischen Instrumentes nach den Fig. 1 und 2,
Fig. 6 die Sicherungseinheit gemäß Fig. 5 in einer Seitenansicht, die einen Arretierabschnitt einer männlichen Arretieranordnung der Umlaufsperre erkennen lässt,
Fig. 7 schematisch einen Teilbereich einer weiteren Ausführungsform eines erfindungsgemäßen chirurgischen Instrumentes ähnlich den Fig. 2 oder 3, jedoch mit einer translatorischen Linearführung für einen Arretierabschnitt der Umlaufsperre entlang einer Kreisbogenbahn,
Fig. 8 eine weitere Ausführungsform eines erfindungsgemäßen chirurgischen Instrumentes mit einem rotatorisch gelagerten Arretierabschnitt einer männlichen Arretieranordnung einer Umlaufsperre,
Fig. 9 in vergrößerter perspektivischer Darstellung einen Teilbereich des chirurgischen Instrumentes gemäß Fig. 8 mit der männlichen Arretieranordnung,
Fig. 10 einen Teilbereich eines Greifschenkels des chirurgischen Instrumentes gemäß Fig. 8, dem die männliche Arretieranordnung zugeordnet ist,
Fig. 11 eine weitere Darstellung analog Fig. 10,
Fig. 12 eine Schnittdarstellung des Teilbereichs gemäß Fig. 11 entlang der Schnittlinie XII-XII in Fig. 11,
Fig. 13 den Teilbereich gemäß Fig. 10, jedoch mit dem Arretierabschnitt in seiner Funktionsstellung,
Fig. 14 in einer Seitenansicht Funktionskomponenten einer Zwangssteuereinrichtung für die Arretieranordnung gemäß den Fig. 8 bis 13,
Fig. 15 eine Schnittdarstellung entlang der Schnittlinie XV-XV in Fig. 14,
Fig. 16a bis 16d in unterschiedlichen Ansichten eine Funktionskomponente der Zwangssteuereinrichtung für die Umlaufsperre gemäß den Fig. 8 bis 15,
Fig. 17a bis 17d in unterschiedlichen Ansichten eine weitere Funktionskomponente der Zwangssteuereinrichtung für die Umlaufsperre gemäß den Fig. 8 bis 15,
Fig. 18a bis 18d in unterschiedlichen Darstellungen eine weitere Funktionskomponente der Zwangssteuereinrichtung für die Umlaufsperre gemäß den Fig. 8 bis 15,
Fig. 19a bis 19d ein manuelles Betätigungselement für die Zwangssteuereinrichtung der Umlaufsperre gemäß den Fig. 8 bis 15,
Fig. 20 eine weitere Ausführungsform eines erfindungsgemäßen chirurgischen Instrumentes mit einer vereinfachten, rotatorisch wirksamen männlichen Arretieranordnung,
Fig. 21 in vergrößerter Darstellung einen Teilbereich des chirurgischen Instrumentes gemäß Fig. 20 mit der männlichen Arretieranordnung,
Fig. 22 und 23 unterschiedliche Funktionslagen der Arretieranordnung gemäß Fig. 21,
Fig. 24 eine männliche Arretieranordnung gemäß einer geringfügig modifizierten Ausführungsform der Erfindung und
Fig. 25 und 26 in unterschiedlichen Darstellungen eine weitere Ausführungsform einer erfindungsgemäßen männlichen Arretieranordnung für ein chirurgisches Instrument gemäß einer erfindungsgemäßen Ausführungsform.

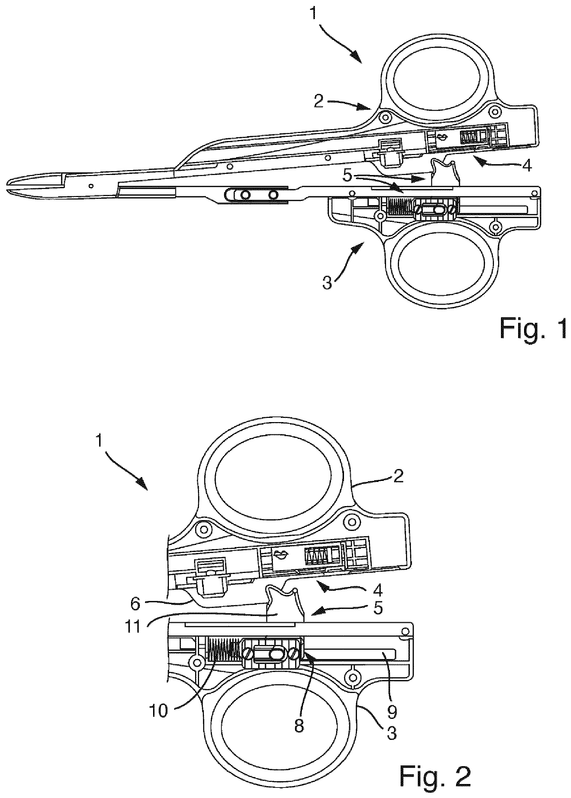

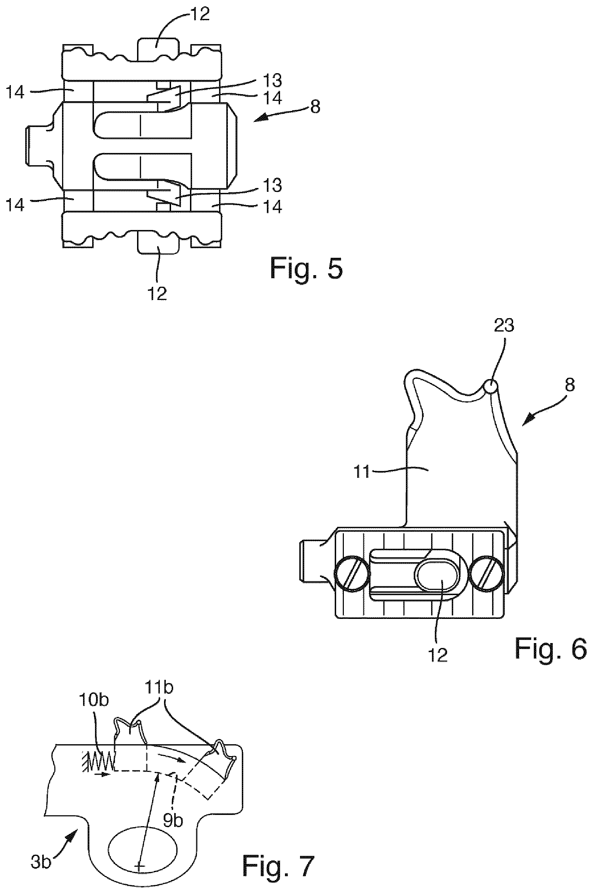

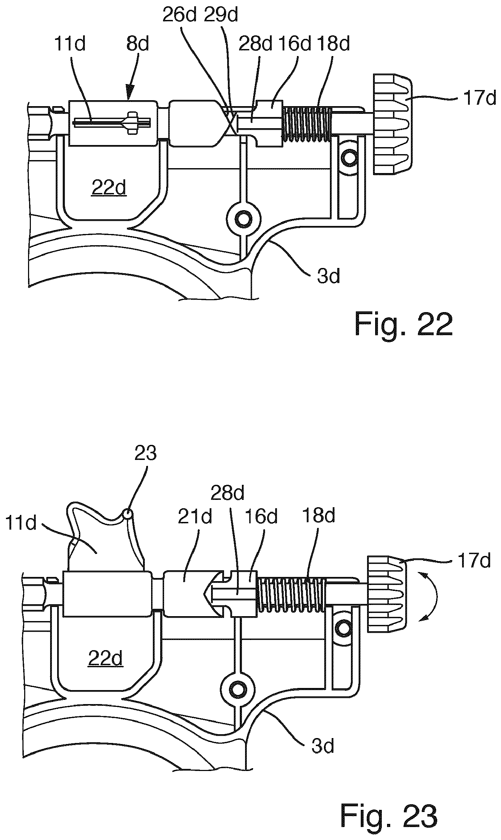

Anhand der Fig. 1 bis 26 werden nachfolgend verschiedene Ausführungsformen erfindungsgemäßer chirurgischer Instrumente 1 bis 1f beschrieben, wobei funktionsgleiche Abschnitte oder Komponenten gleiche Bezugszeichen unter Hinzufügung des jeweiligen Buchstabens a bis f, je nach Ausführungsform, aufweisen. Bei allen Ausführungsformen gemäß den Fig. 1 bis 26 sind zwei relativ zueinander in einer gemeinsamen Schwenkebene schwenkbewegliche Greifschenkel 2, 3 (zuzüglich der entsprechenden Buchstabenvariante) vorgesehen, die für eine komplementäre Bewegung zweier Klemmbacken relativ zueinander schwenkbeweglich gelagert sind. Den beiden Greifschenkeln 2, 3 ist eine Umlaufsperre zugeordnet, die eine lösbare Arretierung der beiden Greifschenkel 2, 3 relativ zueinander ermöglicht, um insbesondere die beiden Klemmbacken in einer Klemmstellung zu halten. Die Umlaufsperre weist bei allen Ausführungsformen eine weibliche Arretieranordnung 4 sowie eine männliche Arretieranordnung 5 (zuzüglich der buchstabengemäß unterscheidbaren Varianten) auf. Bei allen dargestellten und nachfolgend beschriebenen Ausführungsformen ist die weibliche Arretieranordnung 4, 4a, 4c, 4d identisch gestaltet zu der weiblichen Arretieranordnung, wie sie in der DE 10 2016 118 199 A1, dort insbesondere anhand der Fig. 6 bis 11, dargestellt und näher beschrieben ist. Die weibliche Arretieranordnung weist demzufolge einen in Längsrichtung des Greifschenkels 2, 2a, 2c, 2d linearbeweglich und federkraftunterstützt verfahrbaren Arretierschlitten auf, der mit einer Steuerkulisse versehen ist, in die ein Steuernocken eines Arretierabschnittes 11 bis 11f einer männlichen Arretieranordnung eingreift. Der Arretierschlitten ist entlang einer Längsrichtung des Greifschenkels 2d federkraftbelastet linearbeweglich verschiebbar. Das Zusammenwirken des Steuernockens des Arretierabschnittes der männlichen Arretieranordnung 5 bis 5f mit der Steuerkulisse des Arretierschlittens der weiblichen Arretieranordnung 4 bis 4d erfolgt nach Art einer Herzkurve analog des Kugelschreiberprinzips, wobei der Steuer- oder Rastnocken 23 des Arretierabschnittes der männlichen Arretieranordnung 5 bis 5f unter Querverlagerung des Arretierschlittens an entsprechenden Steuerkonturen der Steuerkulisse entlanggleitet, bis der Steuer- oder Rastnocken in einer Rastaufnahme der Steuerkulisse zur Anlage kommt. In dieser Stellung ist die Umlaufsperre arretiert. Diese Stellung erreicht der Arretierabschnitt der männlichen Arretieranordnung durch manuelles Zusammendrücken der Greifschenkel 2, 3 gegeneinander. Durch erneutes kurzes Zusammendrücken gelangt der Steuer- oder Rastnocken des Arretierabschnittes der männlichen Arretieranordnung 5 in die Freigabespur der Steuerkulisse, wodurch der Arretierabschnitt wieder freikommt und die beiden Greifschenkel 2, 3 voneinander getrennt und auseinandergeschwenkt werden können. Hierdurch entfernen sich zwangsläufig auch die beiden Klemmbacken voneinander, wodurch diese aus der Klemmstellung wieder in die Freigabestellung überführt werden.

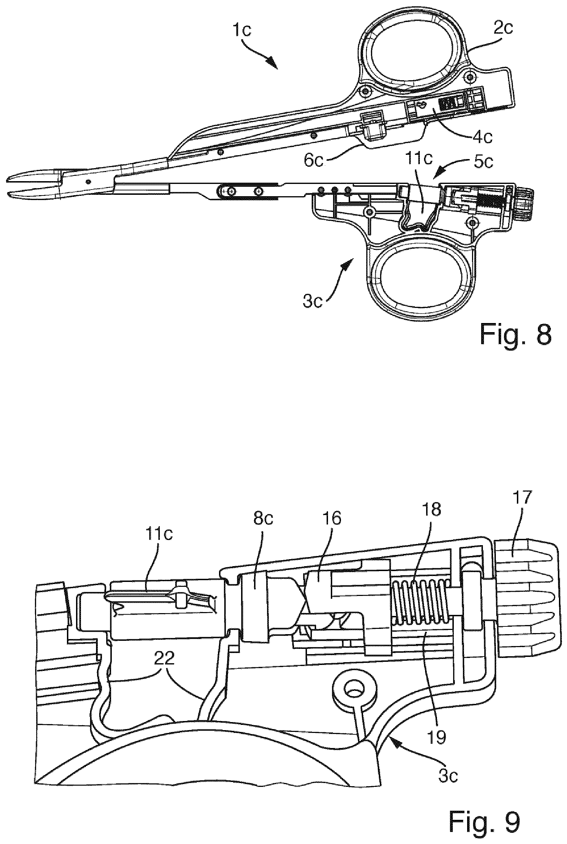

Die nachfolgend beschriebenen Ausführungsformen unterscheiden sich ausschließlich durch die Gestaltung und Steuerung der männlichen Arretieranordnung voneinander. Dabei zeigen die chirurgischen Instrumente gemäß den Fig. 1 bis 7 Ausführungsformen, bei denen die Arretierabschnitte der männlichen Arretieranordnungen translatorisch beweglich gelagert sind zwischen ihren unterschiedlichen Endlagen. Die Ausführungsformen gemäß den Fig. 8 bis 26 hingegen offenbaren Ausführungsformen von chirurgischen Instrumenten, bei denen der jeweilige Arretierabschnitt der männlichen Arretieranordnung jeweils rotatorisch zwischen seinen Endlagen, das heißt einer Ruhestellung und einer Funktionsstellung, verlagerbar sind. Nachfolgend werden die einzelnen Ausführungsformen im Detail beschrieben. Alle Ausführungsformen weisen im Bereich der einander zugewandten Seiten der Greifschenkel Führungsstege 6 auf (siehe insbesondere Fig. 2), die eine Stabilisierung der Umlaufsperre gegen Querkrafteinflüsse bewirken.

Das chirurgische Instrument 1 nach den Fig. 1, 2 und 4 bis 6 weist eine männliche Arretieranordnung 5 auf, bei der ein steg- oder schwertförmig von dem Greifschenkel 3 abragender Arretierabschnitt 11 mittels einer Steueranordnung 8 zwischen einer Funktionsstellung gemäß den Fig. 1 und 2 und einer eine Ruhestellung definierenden Endlage verlagert werden kann. Die Steueranordnung 8 weist einen Steuerschlitten auf, an dem der Arretierabschnitt 11 mit seinem Steuer- oder Rastnocken 23 (Fig. 6) fest angeordnet ist. Der Steuerschlitten ist mithilfe säulenartiger Führungen 14 in einer geradlinigen Linearführung 9 in Längsrichtung des Greifschenkels 3 geführt, die parallel zu einem dem gegenüberliegenden Greifschenkel 2 zugewandten Randbereich verläuft. Die Linearführung 9 wird durch Führungsnuten in zwei Gehäuseschalen des Greifschenkels 3 gebildet, wobei anhand der Fig. 1 und 2 der besseren Übersichtlichkeit halber eine obere Gehäuseschale weggelassen wurde. Zudem ist für den Arretierabschnitt 11 in nicht näher dargestellter Weise eine Führungsnut in dem dem gegenüberliegenden Greifschenkel 2 zugewandten Randbereich vorgesehen, deren Ränder den Arretierabschnitt 11 beidseitig flankieren, um diesen gegen auftretende Querkräfte abzustützen. Der Steuerschlitten der Steueranordnung 8 ist permanent durch eine Federeinrichtung 10, vorliegend in Form einer Schraubendruckfeder, in Richtung seiner die Ruhestellung bildenden Endlage, das heißt zu einem proximalen Ende des Greifschenkels 3 hin, federbelastet. Bei einer nicht dargestellten Ausführungsform der Erfindung ist die Federeinrichtung in entgegengesetzter Richtung wirksam, d.h. in Richtung der die Funktionsstellung bildenden Endlage. Um den Arretierabschnitt 11 in der Funktionsstellung gemäß den Fig. 1 und 2 arretieren zu können, weist die Steueranordnung 8 Sicherungsmittel in Form einer Rastanordnung auf, die durch zwei elastisch bewegliche Rastnasen 13 und komplementäre Rastaussparungen 14 in dem Greifschenkel 3 gebildet sind. Die Rastaussparungen 14 sind ortsfest im Bereich der Linearführung 9 in dem Greifschenkel 3 vorgesehen. Die Rastaussparungen 14 sind derart positioniert, dass der Arretierabschnitt 11 sich nach dem Einrasten der Rastnasen 13 in die Rastaussparungen 14 in der Funktionsstellung befindet, in der er mit der weiblichen Arretieranordnung 4 bei einem Zusammendrücken der Greifschenkel 2 und 3 zusammenwirken kann, um die Arretierung der Umlaufsperre zu erzielen. Der Steueranordnung 8 sind auf gegenüberliegenden Seiten des Greifschenkels 3 - in der Darstellung gemäß Fig. 2 im Bereich einer Oberseite und im Bereich einer Unterseite - nicht näher bezeichnete Schiebeelemente zugeordnet, die an dem Steuerschlitten der Steueranordnung 8 fest angeordnet sind. Unter der festen Anordnung kann eine einstückige Anformung oder eine kraft- oder formschlüssige oder eine stoffschlüssige Verbindung verstanden werden. Die Schiebeelemente sind Teile einer manuell bedienbaren Betätigungseinrichtung, die zum Lösen der Sicherungsmittel vorgesehen ist. Den Schiebeelementen sind Betätigungsnocken 12 zugeordnet, die elastisch beweglich angeordnet sind und mit den Rastnocken 13 des Steuerschlittens 8 zusammenwirken können. Durch einfaches, insbesondere gleichzeitiges Drücken der Betätigungsnocken 12 nach innen werden zwangsläufig auch die Rastnocken 13 nach innen gedrückt, wodurch diese aus den Rastaussparungen 14 freikommen und die Federeinrichtung 10 den Steuerschlitten zwangsläufig in die proximale Endlage der Linearführung 9 drücken kann. Die Federeinrichtung 10 ist in ihrer wirksamen Länge so auf den maximalen Weg, den die Steueranordnung 8 innerhalb der Linearführung 9 zurücklegen kann, abgestimmt, dass der Steuerschlitten der Steueranordnung 8 in jeder Stellung durch die Federeinrichtung 10 federbelastet ist. Eine Rückführung der Steueranordnung 8 aus der proximalen, die Ruhestellung bildenden Endlage in die in den Fig. 1 und 2 dargestellte, die Funktionsstellung bildende Endlage erfolgt manuell durch Ergreifen der Schiebeelemente und Verschieben der Steueranordnung 8 entgegen der Federkraft der Federeinrichtung 10, bis die Steueranordnung 8 mit den Rastnocken 13 wieder die Rastaussparungen 14 erreicht hat, in denen die Rastnocken 13 selbsttätig in die Rastaussparungen 14 einrasten und so den Arretierabschnitt 11 in dieser Funktionsstellung sichern.

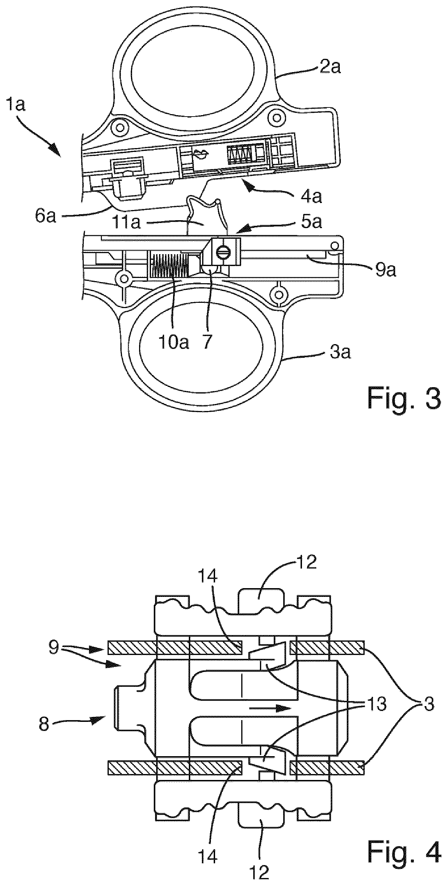

Bei dem chirurgischen Instrument 1a gemäß Fig. 3 sind funktionsgleiche Abschnitte und Komponenten mit gleichen Bezugszeichen, aber unter Hinzufügung des Buchstabens a versehen. Zur Vermeidung von Wiederholungen wird daher ergänzend auf die Ausführungen zu der Ausführungsform gemäß den Fig. 1, 2 und 4 bis 6 verwiesen. Nachfolgend wird lediglich auf die Unterschiede des chirurgischen Instrumentes 1a zu der zuvor beschriebenen Ausführungsform eingegangen. Auch bei dieser Ausführung sind Schiebeelemente als Teile einer manuell bedienbaren Betätigungseinrichtung vorgesehen.

Wesentlicher Unterschied bei dem chirurgischen Instrument 1a ist es, dass statt eines formschlüssig wirksamen Sicherungsmittels eine kraftschlüssig wirksame Sicherung für den Arretierabschnitt 11a in der die Funktionsstellung definierenden Endlage vorgesehen ist. Hierzu weist der Greifschenkel 3a an der die Funktionsstellung definierenden Endlage im Bereich der Linearführung 9a eine kleine schalenartige Vertiefung 7 auf. Die nicht näher bezeichnete Steueranordnung mit ihrem Steuerschlitten, der den Arretierabschnitt 11a trägt, und die durch die Federeinrichtung 10a druckbeaufschlagt ist, weist eine komplementäre, noppenförmige Profilierung auf, die reibschlüssig in die Vertiefung 7 eintauchen kann und so eine kraftbegrenzte Sicherung des Arretierabschnittes 11a in der Funktionsstellung bewirkt. Die durch diese reibschlüssige Sicherung bewirkte Rückhaltekraft ist höher als die Druckkraft der Federeinrichtung 10a, gleichzeitig aber geringer als eine manuell aufbringbare Kraft, um durch Ergreifen der Schiebeelemente der Betätigungseinrichtung den Arretierabschnitt 11a in Richtung der proximalen Endlage verschieben zu können. Die Überführung des Arretierabschnittes 11a nach dem Überdrücken der reibschlüssigen Sicherung in der Funktionsstellung bis in die proximale Endlage erfolgt zwangsläufig durch eine entsprechende Druckbelastung der Federeinrichtung 10a analog der zuvor beschriebenen Ausführungsform.

Bei der Ausführungsform gemäß Fig. 7 ist der Arretierabschnitt 11b der männlichen Arretieranordnung ebenfalls translatorisch verschiebbar. In nicht näher dargestellter Weise ist dem Arretierabschnitt 11b für die in Fig. 7 links dargestellte Funktionsstellung eine kraft- oder formschlüssig wirksame Sicherung zugeordnet, wie sie anhand der zuvor beschriebenen Ausführungsformen offenbart wurde. Zur Vermeidung von Wiederholungen wird diesbezüglich somit ebenfalls auf die entsprechende Offenbarung zu den vorhergehenden Ausführungsformen verwiesen. Maßgeblicher Unterschied der Ausführungsform gemäß Fig. 7 ist es, dass die Linearführung 9b in dem Greifschenkel 3b nicht geradlinig, sondern vielmehr entlang eines Kreisbogens in der Schwenkebene des Greifschenkels 3b erstreckt ist. Ein Mittelpunkt dieses Kreisbogens ist in Fig. 7 schematisch dargestellt. Ein dem gegenüberliegenden Greifschenkel zugewandter Randbereich des Greifschenkels 3b weist an dem proximalen Ende eine nicht näher bezeichnete Tasche auf, in die der Arretierabschnitt 11b bei seiner Überführung in die proximale Endlage eintauchen kann. In dieser proximalen Endlage ist der Arretierabschnitt 11b innerhalb der Außenkonturen des Greifschenkels 3b angeordnet, so dass sein schwert- oder stegförmig abragender und mit dem Steuer- oder Rastnocken versehener Bereich nicht mehr über den Randbereich des Greifschenkels 3b hinausragt. Eine Bedienperson kann insbesondere mit einem Handschuh somit in der Ruhelage des Arretierabschnittes 11b nicht an dem Arretierabschnitt 11b hängen bleiben. Auch bei dieser Ausführungsform ist die Federeinrichtung 10b Teil der Steueranordnung, die den Arretierabschnitt 11b nach dem Lösen aus seiner Funktionsstellung zwangsläufig in die die Ruhestellung definierende Endlage überführt.

Bei den Ausführungsformen gemäß den Fig. 8 bis 26 wird der jeweilige Arretierabschnitt 11c bis 11f rotatorisch zwischen seinen beiden Endlagen verlagert, die die Funktionsstellung bzw. die Ruhestellung definieren. Bei dem chirurgischen Instrument 1c gemäß den Fig. 8 bis 19d ist dem Arretierabschnitt 11c zudem eine nachfolgend näher beschriebene Zwangssteuereinrichtung zugeordnet, die derart gestaltet ist, dass der Arretierabschnitt 11c nicht in einer Zwischenlage verbleiben kann, sondern zwangsläufig federkraftbeaufschlagt immer in eine der beiden Endlagen verschwenkt. Der Zwangssteuereinrichtung gemäß den Fig. 8 bis 19d ist zudem in nachfolgend beschriebener Weise eine Verzögerungseinheit zugeordnet, die den Arretierabschnitt 11c erst bei größeren Steuerausschlägen beaufschlagt. Dadurch ist gewährleistet, dass der Arretierabschnitt 11c insbesondere beim Auftreten von Querkräften in seiner Endlage verbleibt. Bei allen Ausführungsformen sind funktionsgleiche Teile und Abschnitte mit gleichen Bezugszeichen unter Hinzufügung unterscheidungskräftiger Kleinbuchstaben versehen. Zur Vermeidung von Wiederholungen wird jeweils ergänzend auf die anderen beschriebenen Ausführungsformen verwiesen.

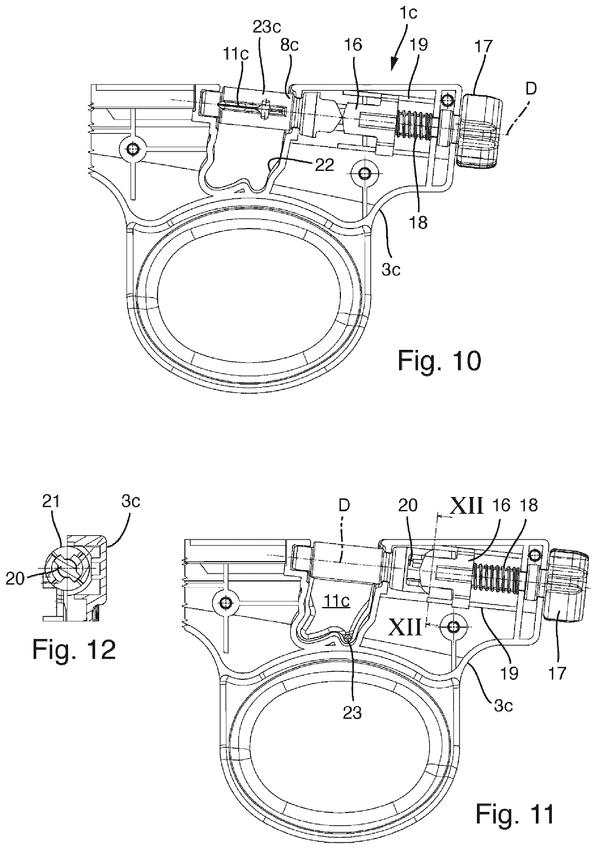

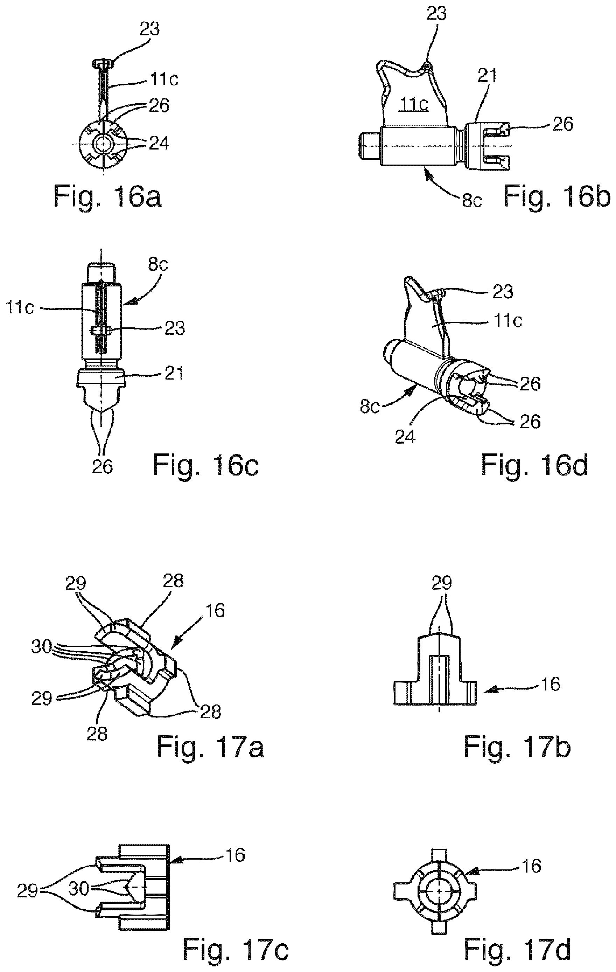

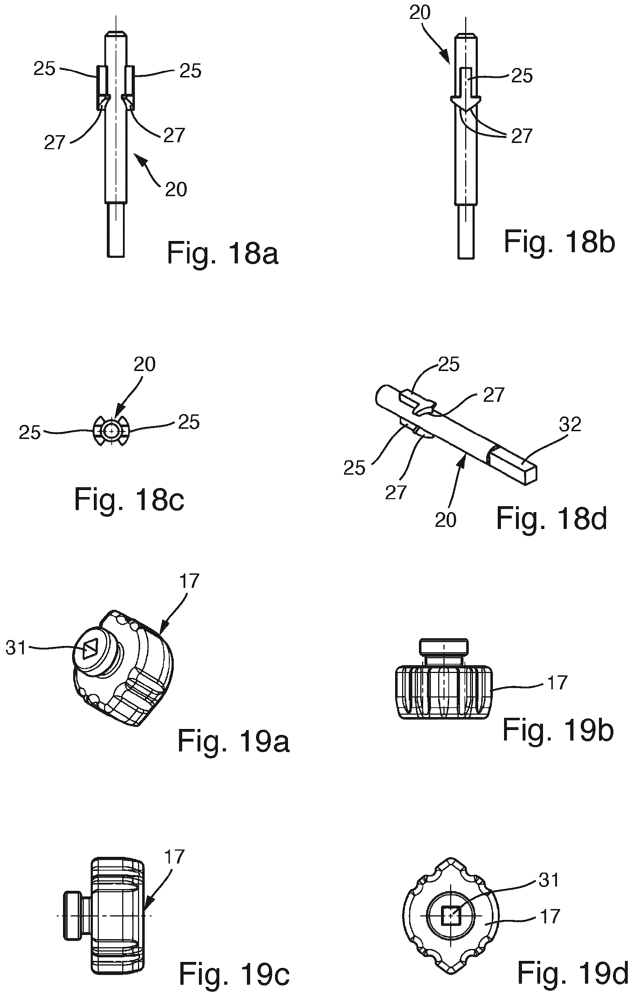

Die männliche Arretieranordnung des chirurgischen Instrumentes 1c gemäß den Fig. 8 bis 19d ist mittels einer Drehlagerung rotatorisch um eine Drehachse D drehbeweglich in dem Greifschenkel 3c gelagert, wobei die Drehachse D zumindest weitgehend in Längsrichtung des Greifschenkels 3c verläuft, wie anhand der Fig. 10 und 11 erkennbar ist. Eine Steueranordnung 8c bildet einen rotierbaren Körper, der in entsprechenden Lagerstellen des Greifschenkels 3c (siehe insbesondere Fig. 9) drehbar gelagert ist. Die Lagerstellen flankieren einen Durchtritt 22 des Greifschenkels 3c, der sowohl in einer nicht dargestellten oberen Gehäuseschale als auch in einer in Fig. 9 gut erkennbaren unteren Gehäuseschale des Greifschenkels 3c ausgebildet ist. Der Durchtritt 22 ist zu dem benachbarten Greifschenkel 2c hin offen, wie anhand der Fig. 8 erkennbar ist. Von dem rotatorischen Körper der Steueranordnung 8c ragt radial der steg- oder schwertförmige Arretierabschnitt 11c ab, der einstückig an dem Körper der Steueranordnung 8c angeformt ist. Der Arretierabschnitt 11c weist den Steuer- oder Rastnocken 23c auf, der bezüglich seiner Funktion zuvor bereits beschrieben worden war. Aufgrund des Durchtrittes 22 ist der Arretierabschnitt 11c somit um 360° um die Drehachse D durch den Griffschenkel 3c hindurch rotierbar. Der Steueranordnung 8c ist eine nachfolgend näher beschriebene Zwangssteuereinrichtung zugeordnet, die dazu dient, den Arretierabschnitt 11c immer in eine seiner beiden Endlagen zu führen, das heißt der zum gegenüberliegenden Greifschenkel 2c gewandten Funktionsstellung oder der in dem Durchtritt 22 aufgenommenen Ruhestellung, und dort zu halten. Hierfür sind mehrere Funktionskomponenten vorgesehen, die anhand der Fig. 16a bis 19d näher dargestellt sind. Die Steueranordnung 8c weist an ihrem proximalen Ende ein Steuermaul 21 auf, das stirnseitig mit in Umfangsrichtung wendelförmig ansteigenden bzw. abfallenden Steuerkonturen 26 versehen ist, wie insbesondere anhand der Fig. 16a bis 16d erkennbar ist. Das Steuermaul 21 weist zudem innen liegend insgesamt vier in Umfangsrichtung zueinander versetzte Steueranschläge 24 auf. Die wendelförmigen Steuerkonturen 26 sind an zwei einander radial gegenüberliegenden und sich axial zum proximalen Ende des Greifschenkels 3c erstreckenden Fortsätzen stirnseitig vorgesehen, wobei die Steuerkonturen 26 in gleichen Winkeln, aber gegensinnig zueinander ansteigen, so dass an den gegenüberliegenden Fortsätzen sich jeweils eine mittige Bugspitze ergibt. Das Steuermaul 21 weist zudem koaxial zur Drehachse D innenseitig eine zylindrische Aufnahme auf, in der ein zylindrisches distales Stirnende einer Betätigungsstange 20 (siehe Fig. 18a bis 18d) relativ drehbeweglich gelagert ist. Ein proximales Stirnende der Betätigungsstange 20 ist mit einem Vierkant 32 versehen, auf den ein Betätigungselement 17 in Form eines Drehrades drehschlüssig aufsteckbar ist (Fig. 19a bis 19d). Bei nicht dargestellten weiteren Ausführungsformen der Erfindung ist das Stirnende mit einer anderen, rotationsunsymmetrischen Gestaltung versehen, auf das das Betätigungselement drehschlüssig aufsteckbar ist. Das Betätigungselement 17 weist eine komplementäre vierkantförmige Aussparung 31 auf, mittels der das drehschlüssige Aufstecken des Betätigungselements 17 auf das proximale Stirnende der Betätigungsstange 20 und damit auf den Vierkant 32 gewährleistet ist. Wie anhand der Fig. 8 bis 11 und 13 erkennbar ist, ist das Betätigungselement 17 koaxial zur Drehachse D im Bereich einer Lagerstelle an einem proximalen Ende des Greifschenkels 3c in dem Greifschenkel 3c drehbar gelagert.

Der Steueranordnung ist zudem ein Steuerschieber 16 (siehe Fig. 17a bis 17d) zugeordnet, der in einer Linearführung 19, die in den Zeichnungen lediglich angedeutet ist, koaxial zur Drehachse D translatorisch verschiebbar gelagert ist. Der Steuerschieber 16 ist permanent durch eine Federeinrichtung 18 in Form einer Schraubendruckfeder beaufschlagt, die die Betätigungsstange 20 umgibt, sich proximal im Bereich der Lagerstelle für das Betätigungselement 17 ortsfest abstützt, und die sich distal an einer Stirnseite des Steuerschiebers 16 abstützt, um diesen permanent in Längsrichtung der Drehachse D mit einer Druckfederkraft zu beaufschlagen. Der Steuerschieber 16 umschließt die Betätigungsstange 20 hohlzylindrisch und ist demzufolge relativ zu der Betätigungsstange 20 begrenzt linearbeweglich verschiebbar. Die Betätigungsstange 20 wiederum ist innerhalb des Steuerschiebers 16 koaxial zur Drehachse D drehbar gelagert. Alle Funktionskomponenten, das heißt die Steueranordnung 8c mit dem Steuermaul 21, der Steuerschieber 16, die Betätigungsstange 20, die Federeinrichtung 18 und das Betätigungselement 17, sind durch einfaches Zusammenstecken relativ zueinander betriebsfertig montierbar, ohne dass zusätzliche Fixiermittel benötigt werden. Das Betätigungselement 17 und die Betätigungsstange 20 bilden eine manuell bedienbare Betätigungseinrichtung im Sinne der Erfindung. Die Federeinrichtung 18 weist je nach Ausführung eine lineare oder eine progressive Federkennlinie auf, abhängig davon, wie die übrigen Funktionskomponenten bezüglich ihrer Zwangssteuerfunktion und ihrer Verzögerungsfunktion für den Arretierabschnitt 11c zusammenwirken.

Wie anhand der Fig. 18a bis 18d erkennbar ist, weist die Betätigungsstange 20 im Bereich ihres zylindrischen Außenumfangs auf gegenüberliegenden Seiten jeweils einen einstückig angeformten Steueraufsatz auf, wobei jeder der beiden Steueraufsätze durch jeweils einen in Längsrichtung erstreckten Steuernocken 25 sowie einen proximal an den Steuernocken 25 anschließenden Steuerkeil 27 gebildet ist, der proximal mit in Umfangsrichtung wendelförmig ansteigenden bzw. gegensinnig abfallenden Steuerkonturen versehen ist. Die gegensinnigen Steuerkonturen bilden eine Bugspitze analog des Steuermauls 21. Die entsprechende Bugspitze ist proximal zu dem Vierkantende 32 der Betätigungsstange 20 gerichtet, so dass beide einander diametral gegenüberliegenden Steueraufsätze parallel zueinander erstreckte, distal ausgerichtete Steuernocken 25 und proximal ausgerichtete Steuerkeile 27 aufweisen.

Der Steuerschieber 16 weist, wie anhand der Fig. 17a bis 17d erkennbar ist, analog zu dem Steuermaul 21 der Steueranordnung 8c zwei maulförmig abragende Steuerfortsätze auf, die stirnseitig mit wendelförmig axial ansteigenden bzw. abfallenden Steuerkonturen 29 versehen sind analog der Steuerkonturen 26 des Steuermauls 21. Auch die Steuerkonturen 29 der Fortsätze des Steuerschiebers 16 bilden jeweils paarweise eine Bugspitze analog dem Steuermaul 21 der Steueranordnung 18. Der Steuerschieber 16 weist zudem in Umfangsrichtung um 90° versetzt sowie axial gegenüber den Fortsätzen mit den Steuernocken 29 zurückgesetzt zwei weitere Paare von Steuernocken 30 auf, die radial auf einer weiter innen liegenden Umfangslinie positioniert sind wie die Steuernocken 29. Auch diese paarweisen Steuernocken 30 bilden gegenüberliegend zueinander jeweils eine Bugspitze, die von einer in Umfangsrichtung axial ansteigenden Steuerkontur und einer in gleicher Umfangsrichtung axial abfallenden Steuerkontur 30 flankiert sind. Diese Steuerkonturen 30 sind radial gesehen auf einer gleichen Umfangslinie vorgesehen wie die Steuerkeile 27 der Betätigungsstange 20.

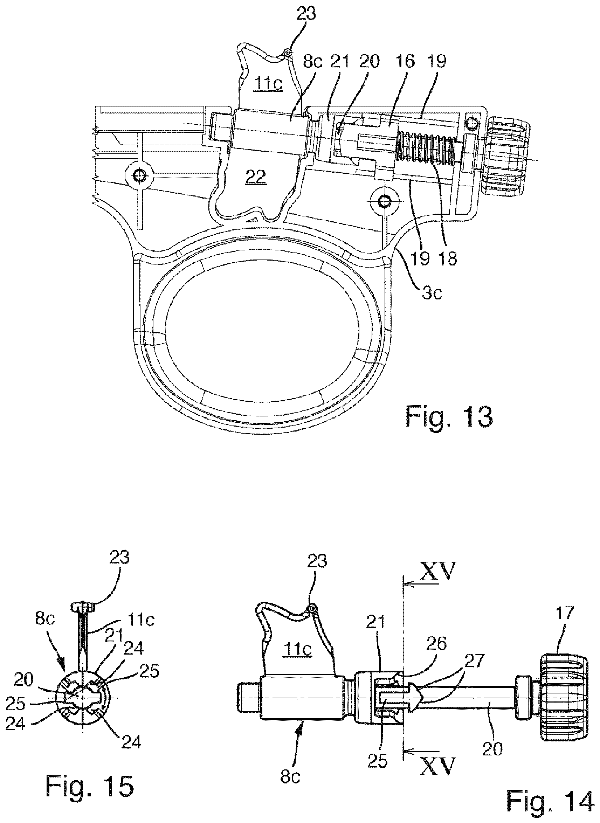

In montiertem Betriebszustand sind die Steuerkonturen der beiden Steuerkeile 27 der Betätigungsstange 20 und die radial innen liegenden Steuerkonturen 30 des Steuerschiebers 16 einander zugewandt und miteinander in Kontakt. Zudem sind die radial außen liegenden Steuerkonturen 29 des Steuerschiebers 16 und die Steuerkonturen 26 des Steuermauls 21 auf gleicher Umfangslinie und je nach Stellung demzufolge miteinander in Kontakt. Die entsprechend diametral gegenüberliegenden, maulartigen Fortsätze des Steuermauls 21 und des Steuerschiebers 16 sind zueinander entgegengerichtet, so dass der Steuerschieber 16 und das Steuermaul 21 axial ineinander eintauchen können nach zuvor erfolgter, zumindest weitgehend rechtwinkliger Verdrehung relativ zueinander. Hierdurch ist der Arretierabschnitt 11c rotatorisch fixiert. Zudem wirken die Steuernocken 25 der Betätigungsstange 20 mit den Steueranschlägen 24 des Steuermauls 21 zusammen. Da die Federeinrichtung 18 permanent eine translatorische Druckkraft auf den Steuerschieber 16 distal zu dem Greifschenkel 3c ausübt, sind der Steuerschieber 16 und das Steuermaul 21 der Steueranordnung 8c permanent miteinander in Kontakt. Dabei ergeben sich zwei stabile Endlagen, in denen die diametral gegenüberliegenden Fortsätze des Steuerschiebers 21 einerseits und des Steuermauls 21 axial ineinandergreifen. Da der Steuerschieber 16 mit seinen maulartigen Fortsätzen lediglich translatorisch verschiebbar ist und demzufolge längs der Drehachse D immer druckfederbeaufschlagt ist, taucht das Steuermaul 21 zwischen diese Fortsätze des Steuerschiebers 16 entweder so ein, dass der Arretierabschnitt 11c in seine Ruhelage gemäß Fig. 11 oder in seine Funktionslage gemäß Fig. 13 verdreht ist. Die beiden Endlagen ergeben sich demzufolge durch jeweils eine Drehung der Steueranordnung 8c und damit des Steuermauls 21 um 180°. Die jeweils aneinanderliegenden Steuerkonturen mit ihren mittigen Bugspitzen gewährleisten in Verbindung mit der permanenten Druckkraft der Federeinrichtung 18, dass sich für den Arretierabschnitt 11c kein instabiler Zustand einstellt. Die Steuerkonturen gleiten in Umfangsrichtung entweder in der einen oder in der anderen Richtung zwangsläufig relativ zueinander ab, bis die maulartigen Fortsätze des Steuermauls 21 und des Steuerschiebers 16 in einer der beiden Endlagen stabil axial ineinandergreifen. Die durch die Steuerkonturen bewirkte Zwangssteuerung wird eingeleitet durch eine Drehbewegung des Betätigungselements 17 in beliebiger Drehrichtung. Da die Steuernocken 25 relativ zu den Steueranschlägen 24 des Steuermauls 21 in einem gewissen Winkelbereich in beiden Drehrichtungen freilaufen, bevor sie einander in Umfangsrichtung kontaktieren, können geringfügige Drehbewegungen des Betätigungselements 17, insbesondere im Bereich zwischen 5° und etwa 85°, keine Aktivierung einer Drehbewegung des Arretierabschnittes 11c auslösen. Erst, wenn das Betätigungselement 17 so weit verdreht wird (um wenigstens etwa 90°), dass die Steuernocken 25 in der einen oder in der anderen Drehrichtung an den Steueranschlägen 24 des Steuermauls 21 anschlagen, wird der Arretierabschnitt 11c entsprechend drehmomentbeaufschlagt und verdreht. Die Drehbewegung des Betätigungselements 17 muss nach dem Einleiten der Drehbewegung auf den Arretierabschnitt 11c nicht zu Ende geführt werden, um den Arretierabschnitt 11c bis in die entsprechende Endlage zu verdrehen. Vielmehr gleiten die entsprechenden Steuerkonturen der beschriebenen Funktionskomponenten nach dem Vorbeigleiten der jeweiligen Bugspitzen in Umfangsrichtung aneinander zwangsläufig aufgrund der permanenten Antriebskraft der Federeinrichtung 18 relativ zueinander ab, bis die andere stabile Endlage zwischen dem Steuerschieber 16 und dem Steuermaul 21 der Steueranordnung 8c erreicht ist. Der Arretierabschnitt 11c kann demzufolge nie in einer instabilen Zwischenlage verbleiben. Vielmehr führt die Zwangssteuereinrichtung immer dazu, dass der Arretierabschnitt 11c entweder in der bereits eingestellten Endlage verbleibt, oder aber in die gegenüberliegende Endlage, das heißt die um 180° verdrehte Endlage, überführt wird.

Bei der Ausführungsform gemäß den Fig. 8 bis 19d können entsprechende Steigungen der innen und außen liegenden Steuerkonturen der maulartigen Fortsätze des Steuerschiebers 16 einerseits und die Steuerkonturen der Steuerkeile 27 der Betätigungsstange 20 sowie die Steuerkonturen 26 des Steuermauls 21 andererseits mit unterschiedlichen Steigungen versehen sein, um ein Verklemmen oder Blockieren durch entsprechende Selbsthemmung der aneinander abgestützten Steuerkonturen zu vermeiden. Die entsprechenden Steigungen können zusätzlich auf die Federkennlinie der Federeinrichtung 18 abgestimmt sein, um eine sichere Überführung in die jeweilige Endlage zu erzielen und bewusst mechanische Geräusche für das Erreichen der Endlage zu provozieren.

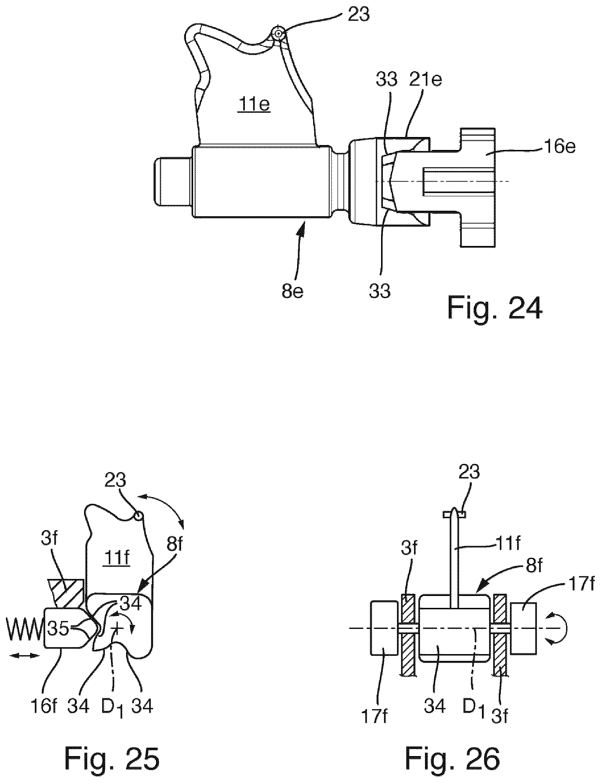

Um in den beiden Endlagen für den Arretierabschnitt 11c eine in Umfangsrichtung spielfreie Positionierung zu ermöglichen, ist es bei einer Variante des chirurgischen Instrumentes 1c, wie sie in Fig. 24 dargestellt ist, vorgesehen, dass das Steuermaul 21 im Bereich der Aussparungen, in die die maulartigen Fortsätze des Steuerschiebers 16e eintauchen, mit Zentrierschrägen 33 versehen ist. Alternativ oder ergänzend können selbstverständlich auch die komplementären Anlageflächen des Steuerschiebers 16e mit Anlaufschrägen versehen sein, um die Zentrierwirkung und demzufolge die in Umfangsrichtung spielfreie Positionierung zwischen dem Steuermaul 21e und dem Steuerschieber 16e zu erreichen.

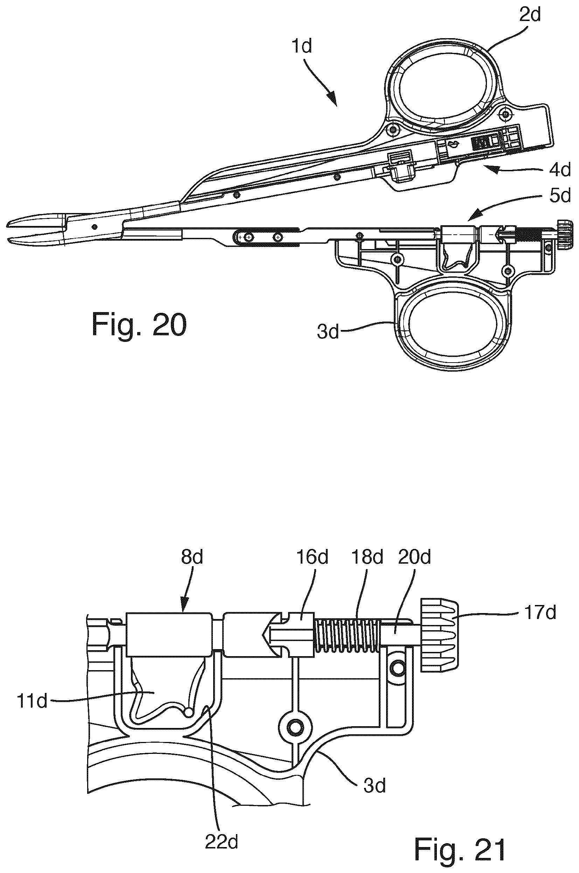

Das chirurgische Instrument 1d gemäß den Fig. 20 bis 23 entspricht weitgehend dem zuvor beschriebenen chirurgischen Instrument 1c gemäß den Fig. 8 bis 19d. Zur Vermeidung von Wiederholungen wird daher ergänzend auf die zuvor beschriebene Ausführungsform verwiesen. Wesentlicher Unterschied ist es, dass der männlichen Arretieranordnung 5d des chirurgischen Instrumentes 1d eine Verzögerungseinheit fehlt, wie sie bei der Ausführungsform gemäß den Fig. 8 bis 19d vorgesehen ist. Die männliche Arretieranordnung 5d ist demzufolge zwar ebenfalls rotatorisch gestaltet, aber einfacher ausgeführt als die Arretieranordnung 5c gemäß den Fig. 8 bis 19d. Bei dem chirurgischen Instrument 1d führt demzufolge eine Verdrehung des Betätigungselements 17d zwangsläufig direkt zu einer Verdrehung des Arretierabschnittes 11d.

Auf den Arretierabschnitt 11d wirkende Drehmomente führen zudem direkt zu einer entsprechenden Relativverdrehung des Arretierabschnittes 11d. Eine Fixierung des Arretierabschnittes in der jeweiligen Endlage fehlt demzufolge bei dieser Ausführungsform im Gegensatz zu der zuvor beschriebenen Ausführungsform. Im Übrigen ist die Zwangssteuereinrichtung zur Verdrehung des Arretierabschnittes 11d zwischen den beiden Endlagen funktional identisch ausgeführt wie bei der zuvor beschriebenen Ausführungsform gemäß den Fig. 8 bis 19d. Funktionsgleiche Teile und Abschnitte sind demzufolge mit gleichen Bezugszeichen, jedoch unter Hinzufügung des Buchstabens d versehen. Zur Vermeidung von Wiederholungen wird demzufolge ergänzend auf die Offenbarung zu den Fig. 8 bis 19d verwiesen.

Die Zwangssteuereinrichtung benötigt lediglich Steuerkonturen im Bereich der maulartigen Fortsätze des Steuerschiebers 16d einerseits und des Steuermauls 21d andererseits, die in der einen oder der anderen Drehrichtung aneinander abgleiten können, um den Arretierabschnitt 11d in die eine Endlage oder in die andere Endlage verdrehen zu können, und um instabile Zwischenlagen des Arretierabschnittes 11d zu vermeiden. Bei dieser Ausführungsform ist der Arretierabschnitt 11d einschließlich des Steuermauls 21d drehfest mit dem als Drehrad ausgebildeten Betätigungselement 17d verbunden. Der Steuerschieber 16d dient lediglich dazu, den Arretierabschnitt 11d in jeweils einer der beiden Endlagen zu fixieren und zudem dazu, auf das Steuermaul 21d durch entsprechendes Abgleiten der Steuerkonturen immer ein Drehmoment in einer der beiden Drehrichtungen aufzubringen, bis die Fortsätze des Steuerschiebers 16d und des Steuermauls 21d axial stabil ineinandergreifen und demzufolge eine weitere Verdrehung in Umfangsrichtung ausgeschlossen ist. Durch ein Drehen des Betätigungselements 17d wird zwangsläufig das Steuermaul 21d verdreht, das den Steuerschieber 16d über die entsprechenden Steuerkonturen gegen die Druckkraft der Federeinrichtung 18d zurückdrängt, bis die Bugspitzen der beiden Funktionskomponenten aneinander vorbeigleiten. Anschließend muss auf das Betätigungselement 17d keine weitere manuelle Drehbewegung mehr eingeleitet werden, da der Steuerschieber 16d über die Steuerschrägen zwangsläufig das Steuermaul 21d in die entsprechend gegenüberliegende, um 180° verdrehte Endlage verdreht und in dieser Endlage in Umfangsrichtung sichert.

Anhand der Fig. 25 und 26 ist schematisch eine weitere Ausführung einer männlichen Arretieranordnung für ein chirurgisches Instrument vorgesehen, das ähnlich der zuvor beschriebenen Ausführungsformen gestaltet ist. Wesentlich bei dieser Ausführungsform ist es, dass der Arretierabschnitt 11f zwar ebenfalls rotatorisch um die Drehachse D₁ gelagert ist, diese Drehachse D₁ jedoch orthogonal zu der Schwenkebene der Greifschenkel ausgerichtet ist. Die Drehachse D der zuvor beschriebenen Ausführungsformen gemäß den Fig. 8 bis 24 hingegen liegt entweder in der Schwenkebene der Greifschenkel 2, 3 oder parallel zu dieser Schwenkebene. Anhand der Darstellungen in den Fig. 25 und 26 ist erkennbar, dass auch diese Ausführung eine Zwangssteuereinrichtung aufweist, wobei ein entsprechender Steuerkörper, mit dem der Arretierabschnitt 11f drehfest verbunden ist, um die Drehachse D₁ begrenzt schwenkbeweglich innerhalb des Greifschenkels 3f gelagert ist. Ein als Steuerelement dienender Steuerkeil 16f ist durch eine Federeinrichtung druckbelastet und in Richtung des Doppelpfeils in Fig. 25 translatorisch begrenzt beweglich gelagert innerhalb des Greifschenkels 3f. Der Steuerkörper 8f, der Teil einer Steueranordnung im Sinne der Erfindung ist, weist im Bereich seiner dem Steuerkeil 16f zugewandten Außenseite mehrere Steuerkonturen 34 auf, die zum einen Rasttaschen zur Sicherung der beiden um 90° zueinander versetzten Endlagen des Arretierabschnittes 11f und zum anderen eine Bugspitze aufweisen, über die eine komplementäre Bugspitze des Steuerkeils 16f hinweg gleitet, um das stabile Einschwenken des Arretierabschnittes 11f in die jeweils andere Endlage zu erzielen. In dem Greifschenkel 3f ist in nicht näher dargestellter Weise eine Aussparung oder Tasche vorgesehen, in die der Arretierabschnitt 11f in seiner die Ruhestellung definieren Endlage eintauchen kann. Diese Endlage ist um 90° verdreht zu der in Fig. 25 und 26 dargestellten und die Funktionsstellung definierenden Endlage des Arretierabschnittes 11f. Koaxial zur Drehachse D₁ sind an gegenüberliegenden Außenseiten des Greifschenkels 3f rotatorische Betätigungselemente 17f, vorliegend in Form von Drehrädern vorgesehen, die jeweils drehschlüssig mit dem Steuerkörper 8f verbunden sind und in den Lagerstellen für den Steuerkörper 8f im Greifschenkel 3f drehbar gelagert sind. Dies ist durch den in Fig. 26 rechts erkennbaren Doppelpfeil verdeutlicht. Der Arretierabschnitt 11f weist in gleicher Weise einen Steuer- oder Rastnocken 23 auf, wie dies bei den zuvor beschriebenen Ausführungsformen der Fall ist. Dieser Steuer- oder Rastnocken 23 wirkt demzufolge in der zuvor beschriebenen Art und Weise mit der nicht dargestellten weiblichen Arretieranordnung zusammen.

## Patentansprüche

1. Chirurgisches Instrument (1, 1a, 1c, 1d) mit
zwei relativ zueinander beweglichen Greifschenkeln (2, 3; 2a, 3a; 2c, 3c; 2d, 3d; 3f), sowie
mit einer Umlaufsperre, die dazu vorgesehen ist, die beiden Greifschenkel (2, 3; 2a, 3a; 2c, 3c; 2d, 3d; 3f) relativ zueinander lösbar zu arretieren,
wobei die Umlaufsperre im Bereich des einen Greifschenkels (2, 2a, 2c, 2d) eine weibliche Arretieranordnung (4, 4a, 4c, 4d) und im Bereich des anderen Greifschenkels (3, 3a, 3b, 3c, 3d, 3f) eine männliche Arretieranordnung (5, 5a, 5c, 5d) aufweist,
wobei die weibliche Arretieranordnung (4, 4a, 4c, 4d) eine mechanische Steuervorrichtung zum Arretieren und Freigeben eines Arretierabschnittes (11, 11a, 11b, 11c, 11d, 11e, 11f) der männlichen Arretieranordnung (5, 5a, 5c, 5d) aufweist,
wobei der männlichen Arretieranordnung (5, 5a, 5c, 5d) eine mechanische Steueranordnung zugeordnet ist, die dazu vorgesehen ist, den Arretierabschnitt (11, 11a, 11b, 11c, 11d, 11e, 11f) der männlichen Arretieranordnung (5, 5a, 5c, 5d) aus einer eine Ruhestellung definierenden Endlage in eine eine Funktionsstellung definierenden Endlage und umgekehrt aus der die Funktionsstellung definierenden Endlage in die die Ruhestellung definierende Endlage zu verlagern,
wobei die Steueranordnung Sicherungsmittel aufweist, die dazu vorgesehen sind, den Arretierabschnitt (11, 11a, 11b, 11c, 11d, 11e, 11f) in beiden Endlagen zu sichern,
wobei wenigstens ein Sicherungsmittel als formschlüssig wirksame Sicherungseinheit ausgeführt ist,
wobei die formschlüssig wirksame Sicherungseinheit spiegelsymmetrisch relativ zu einer Verlagerungsebene der Sicherungseinheit gestaltet ist,
und wobei die Sicherungseinheit insbesondere auf gegenüberliegenden Seiten des Greifschenkels (3) jeweils ein elastisch bewegliches Rastelement (13) aufweist, die mit ortsfesten Rastaussparungen (14) im Bereich des Greifschenkels (3) zusammenwirken.

2. Chirurgisches Instrument nach Anspruch 1, wobei eine manuell bedienbare Betätigungseinrichtung vorgesehen ist, die mit wenigstens einem Sicherungsmittel in Wirkverbindung ist, um das wenigstens eine Sicherungsmittel für eine Freigabe einer Verlagerbarkeit des Arretierabschnittes anzusteuern.

3. Chirurgisches Instrument nach Anspruch 1 oder 2, wobei der Arretierabschnitt (11, 11a, 11b) für eine Verlagerung zwischen den beiden Endlagen längs einer Linearführung translatorisch geführt ist.

4. Chirurgisches Instrument nach Anspruch 3, wobei die Linearführung längs einer Kurvenbahn, insbesondere längs eines Kreisbogens, verläuft.

5. Chirurgisches Instrument nach einem der vorhergehenden Ansprüche, wobei der formschlüssig wirksamen Sicherungseinheit die manuell bedienbare Betätigungseinrichtung (12) zugeordnet ist, um die Sicherungseinheit aus einer Sicherungsstellung in eine Freigabestellung zu überführen.
